# EUROPÄISCHE PATENTANMELDUNG

(11) **EP 0 640 837 A2**
(43) Veröffentlichungstag der Anmeldung: **01.03.1995**
(21) Anmeldenummer: 94113038.7
(22) Anmeldetag: 20.08.1994
(51) Int. Cl.: G01N 33/546, G01N 33/76, G01N 33/80

(54) **Verfahren zur Bestimmung eines Analyten nach dem Agglutinationsprinzip**

(30) Priorität: 26.08.1993 DE 4328684
(71) Anmelder: BOEHRINGER MANNHEIM GMBH, D-68298 Mannheim (DE)
(72) Erfinder: Adema, Enno, Dr., D-82327 Tutzing (DE); Gebert, Ulrike, D-82402 Seeshaupt (DE)

(57) **Zusammenfassung**

Mit einem Verfahren zur Bestimmung eines Analyten aus einer Probenlösung nach dem Agglutinationsprinzip
a) durch Inkubation der Probenlösung mit agglutinierbaren Partikeln, an die analytspezifische Rezeptoren gebunden sind und
b) photometrische Bestimmung der durch die Agglutination verursachten Trübungsänderung der Reaktionslösung als Maß für den Analytgehalt der Probe,
welches dadurch gekennzeichnet ist, daß in Schritt a) zusätzlich zur Probe Analyt zugegeben wird
wird eine Verbesserung der unteren Nachweisgrenze in solchen Tests erreicht.

## Beschreibung

Die Erfindung betrifft ein Verfahren zur Bestimmung eines Analyten nach dem Agglutinationsprinzip sowie ein hierfür geeignetes Reagenz.

Agglutinationsverfahren unter Ausnutzung von immunologischen Bindereaktionen werden häufig zur Bestimmung von niederkonzentrierten Analyten benutzt. Bei Bestimmungen nach dem Agglutinationsprinzip werden durch den zu bestimmenden Analyten in einer immunologischen Reaktion Partikel miteinander zu großen Partikelaggregaten agglutiniert, wobei sich das Streulichtverhalten bzw. die Trübung der Reaktionslösungen ändert. Die quantitative Bestimmung der Analyten erfolgt dann über die Messung der Streulichtintensität (Nephelometrie) oder über die Messung des Intensitätsverlustes von das Reaktionsmedium durchdringendem Licht (Turbidimetrie).

Derartige Verfahren sind dem Fachmann allgemein bekannt und beispielsweise in der DE-A 27 49 956, EP-A 0 356 964, EP-A 0 464 554 und Ann. Clin. Biochem. 29, 22 - 42 (1992), J. Clin. Immunoassay 13, 127 - 131 (1990), Clinical Laboratory Assays Chapter 5, 73 - 95 (1983), eds: Nahamura RM, Ditto WR, Tucher ES beschrieben.

Ein Nachteil von Agglutinationsassays ist, daß oftmals die gewünschte untere Nachweisgrenze nicht erreicht werden kann, weil im unteren Konzentrationsbereich die Sensitivität des Tests zu gering ist.

Zur Verbesserung der Sensitivität wurde bisher vorgeschlagen, möglichst große Latices zu verwenden, die Latices mit einer möglichst großen Menge des korrespondierenden Antikörpers oder Antigens zu sensibilisieren, Antikörper oder Antigene zu verwenden, die eine hohe Spezifität und Affinität zeigen und/oder Latices in möglichst hoher Konzentration zu verwenden (EP A 0 263 731). Ebenso wurde versucht, durch Zusatz von Polyanionen die Genauigkeit der Bestimmung zu verbessern (US-Patent 4,672,045), Harnstoffderivate zuzusetzen (US Patent 4,292,038), halogensubstituierte Essigsäure (US-Patent 4,536,478) oder chaotrope Agentien zuzusetzen (US-Patent 4,362,531).

Aufgabe der vorliegenden Erfindung war es, ein Verfahren zur Bestimmung eines Analyten nach dem Agglutinationsprinzip bereitzustellen, bei dem die Sensitivität im unteren Konzentrationsbereich weiter verbessert und damit Analyt in sehr geringen Konzentrationen nachgewiesen werden kann.

Diese Aufgabe wird gelöst durch ein Verfahren zur Bestimmung eines Analyten aus einer Probenlösung nach dem Agglutinationsprinzip
a) durch Inkubation der Probenlösung mit agglutinierbaren Partikeln, an die analytspezifische Rezeptoren gebunden sind,
b) photometrische Bestimmung der durch die Agglutination verursachten Änderung der Streulichtintensität oder der Trübungsänderung in der Reaktionslösung als Maß für den Analytgehalt der Probe,
dadurch gekennzeichnet, daß im Schritt a) zusätzlich zur Probe Analyt oder eine, sich immunologisch gleich verhaltende Substanz (Analytanaloges) in einer Menge, die dem 2 - 1000fachen der gewünschten unteren Nachweisgrenze entspricht, zugesetzt wird.

Überraschenderweise hat sich gezeigt, daß durch den Zusatz von Analyt oder Analytanalogem (im weiteren als Analyt bezeichnet) die untere Nachweisgrenze deutlich verbessert wird.

Die Menge des zugesetzten Analyten kann in einem weiten Bereich variieren. Sie wird zweckmäßig so gewählt, daß die Meßergebnisse für den zu messenden Konzentrationsbereich eine möglichst große Signaländerung ergeben. Dadurch wird gewährleistet, daß in einem Bereich gemessen wird, in dem die Meßsignale auch für Messungen an der unteren Nachweisgrenze noch ausreichend hoch sind. Die zuzusetzende Höchstmenge an Analyt ist zum einen begrenzt durch die Löslichkeit des Analyten und zum anderen durch den Umstand, daß bei sehr hohen Analytkonzentrationen die Eichkurve flacher wird. In diesem Bereich sind Messungen unvorteilhaft, weil die Sensitivität wieder abnimmt.

Beim D-Dimer Test wird durch Zusatz von D-Dimer zwischen dem 2fachen und 200fachen der Nachweisgrenze die Sensitivität des Tests erheblich gesteigert.

Als agglutinierbare Teilchen können die dem Fachmann bekannten Partikel verwendet werden. Diese Partikel bestehen üblicherweise aus einem inerten Trägermaterial, an welches die analytspezifischen Rezeptoren gebunden sind.

Als Partikel geeignet sind auch Mischungen von Partikeln, an welche verschiedene Antikörper gegen den Analyten gekoppelt sind.

Das inerte Trägermaterial kann beispielsweise Polystyrollatex, Polyvinyltoluol oder Polymethylmethacrylat sein. Die Partikelgröße liegt zweckmäßig zwischen 40 und 1000 nm (vgl. Ann. Clin. Biochem. 29 (1992) 22 - 42).

Analyt und Rezeptor können im Rahmen des erfindungsgemäßen Verfahrens prinzipiell alle Substanzpaarungen (z.B. Biotin/Avidin, Enzym/Substrat, Antikörper/Antigen, Zucker/Lectin) sein, die miteinander bindefähig sind. In diese Definition sind im Rahmen der Erfindung außer immunologisch bindefähigen Substanzpaaren auch solche Substanzpaarungen eingeschlossen, die sich analog verhalten.

Als analytspezifische Rezeptoren, welche an das Trägermaterial gebunden sind, werden vorzugsweise Anti-Analyt Antikörper oder Antikörperfragmente (z. B. Fab',F(ab')₂) verwendet.

Üblicherweise werden eine Vielzahl von solchen Rezeptoren an das inerte Trägermaterial gebunden. Typischerweise werden auf Partikel mit einem Durchmesser von 40 nm etwa 30 Antikörpermoleküle und auf Partikel mit einem Durchmesser von 100 nm etwa 100 Antikörpermoleküle gebunden. Die Mengen sind jedoch von den Beschichtungsbedingungen stark abhängig und an sich unkritisch (Ann. Clin. Biochem. 29 (1992) 22 - 42).

Bei den verwendeten Antikörpern bzw. Antikörperfragmenten kann es sich sowohl um polyklonale als auch um monoklonale Antikörper handeln.

Unter einem Analyt werden Substanzen verstanden, die mindestens zwei Epitope, d. h. Bindungsstellen für den spezifischen Rezeptor besitzen. Bevorzugt wird das erfindungsgemäße Verfahren verwendet, wenn der Analyt ein Protein ist. Der zu bestimmende Analyt kann in einer Körperflüssigkeit, wie Plasma, Serum, Urin, Speichel oder ähnliches oder in einer geeigneten Pufferlösung enthalten sein. Das erfindungsgemäße Verfahren ist beispielsweise besonders geeignet zur Bestimmung von CRP, IgGAM, Ferritin, Albumin oder HCG (Ann. Clin. Biochem. 29, 22 - 42 (1992)).

Für den Fall, daß der Analyt selbst ein Antikörper ist, kann als spezifischer Rezeptor das mit dem Antikörper reagierende Antigen oder aber ein gegen diesen Antikörper gerichteter Anti-Antikörper eingesetzt werden.

Die Messung der Trübung beim erfindungsgemäßen Verfahren kann mit geeigneten Geräten sowohl nephelometrisch als auch turbidimetrisch erfolgen. Die Bestimmung der Konzentration des Analyten in der Reaktionsmischung erfolgt dann durch Vergleich mit einem Standard bekannter Analytkonzentration.

Unter einem Reagenz zur Durchführung des erfindungsgemäßen Verfahrens ist eine Zusammensetzung zu verstehen, die zusätzlich zu den für das Verfahren erforderlichen agglutinierbaren Partikeln Analyt in einer Menge enthält, die der 2 - 1000fachen Menge der gewünschten unteren Nachweisgrenze entspricht.

Die Erfindung wird durch die folgenden Beispiele/Abbildungen erläutert:
Fig. 1 zeigt die Eichkurve für die Bestimmung von D-Dimer (Beispiel 1)
   (a: ohne D-Dimer in R1, b: mit 0,64 µg/ml D-Dimer in R₁)
Fig. 2 zeigt die Eichkurve für die Bestimmung von hCG (Beispiel 2)
   (a: ohne hCG in R₁, b: mit 75 mIU/ml hCG in R₁)

### Beispiel 1

### Bestimmung von D-Dimer

### Reagenz 1:

100 mmol/l Glycin pH 8,3
150 mmol/l NaCl
0,15 % PEG 35000

### Reagenz 2:

0,2 % monoklonale Antikörper gegen D-Dimer, gebunden an Chlormethyllatex in 100 mmol/l Glycinpuffer, pH 8,3.

20 µl Probe, 225 µl Reagenz 1 und 50 µl Reagenz 2 werden zusammengegeben, bei 37°C 5 Minuten inkubiert und die Extinktionsdifferenz unmittelbar nach dem Mischen und 5 Minuten später gemessen. Anhand einer Standardkurve wird aus der gemessenen Extinktionsdifferenz die Konzentration ermittelt. Tabelle 1 zeigt die Ergebnisse für den Fall, daß Reagenz 1 unverändert verwendet wird und in Reagenz 1 0,2, 0,35 µg/ml und 0,64 µg/ml D-Dimer (Analyt) zugesetzt wird. Es zeigt sich, daß durch Zusatz von Analyt die gewünschte Nachweisgrenze erreicht wird.

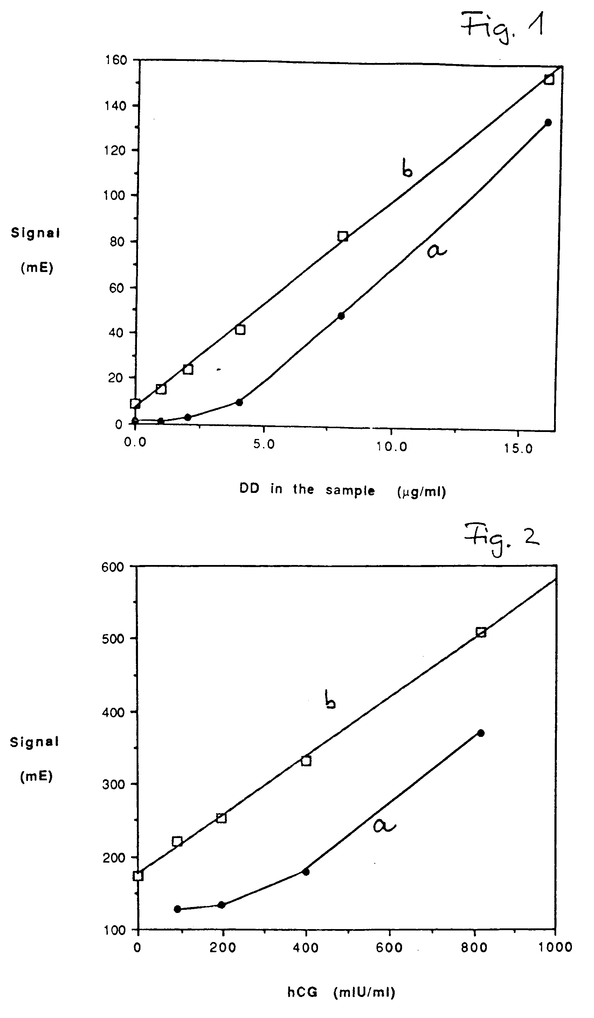

Die Ergebnisse zeigen Fig. 1 und Tabelle 1

**Tabelle I**

| Sensitivität im unteren Meßbereich | | | |
|---|---|---|---|
| D-dimer in Probe (µg/ml) | D-dimer in R1 | | |
| | 0 µg/ml | 0.2 µg/ml | 0.35 µg/ml |
| 0.25 | - 0.002 | 0.006 | 0.012 |
| 0.5 | 0.003 | 0.008 | 0.015 |
| 1 | 0.004 | 0.025 | 0.054 |
| 2 | 0.014 | 0.072 | 0.128 |
| 5 | 0.129 | 0.279 | 0.360 |

### Beispiel 2:

### Bestimmung von HCG

### Reagenz 1:

190 mmol/l Tris-Puffer pH 8,0
0,5 % Rinderserumalbumin
O,1 % Na-azid

### Reagenz 2:

200 mmol/l Glycinpuffer pH 7,5
0,1 % Na-azid
0,2 % mit Anti-hCG-Antikörpern (Fab'₂-Fragmente) beschichtete Latices
50 µl Probe, 330 µl Reagenz 1 und 50 µl Reagenz 2 werden mit/ohne Zusatz von 75 mIU/ml hCG zusammengegeben und, wie in Beispiel 1 beschrieben, der Analyt in der Probe bestimmt. Die Ergebnisse zeigt Fig. 2.

## Patentansprüche

1. Verfahren zur Bestimmung eines Analyten aus einer Probenlösung nach dem Agglutinationsprinzip
a) durch Inkubation der Probenlösung mit agglutinierbaren Partikeln, an die analytspezifische Rezeptoren gebunden sind,
b) photometrische Bestimmung der durch die Agglutination verursachten Trübungsänderung der Reaktionslösung als Maß für den Analytgehalt der Probe,
dadurch gekennzeichnet, daß in Schritt a) zusätzlich zur Probe Analyt in einer Menge, die der 2 - 1000fachen Menge der gewünschten unteren Nachweisgrenze entspricht, zugegeben wird.

2. Verfahren nach Anspruch 1, dadurch gekennzeichnet, daß als agglutinierbare Partikel Latexpartikel, an die analytspezifische Rezeptoren gebunden sind, verwendet werden.

3. Reagenz zum Nachweis eines Analyten nach dem Agglutinationsprinzip, enthaltend
a) agglutinierbare Partikel, die an analytspezifische Rezeptoren gebunden sind,
b) eine definierte Menge an Analyt in einer Menge, die der 2 - 1000fachen Menge der gewünschten unteren Nachweisgrenze entspricht.

4. Reagenz nach Anspruch 3, dadurch gekennzeichnet, daß die Partikel Latexpartikel sind.
